# EUROPEAN PATENT APPLICATION

(11) **EP 4 130 708 A1**
(43) Date of publication of application: **08.02.2023**
(21) Application number: 22188481.0
(22) Date of filing: 03.08.2022
(51) Int. Cl.: G01N 1/22, H01M 10/48

(54) **ASSEMBLY AND METHOD FOR MONITORING THE EMISSIONS OF ONE OR MORE BATTERIES**

(30) Priority: 06.08.2021 IT 202100021485
(71) Applicant: Denios S.r.l., 16017 Isola del Cantone (GE) (IT)
(72) Inventor: D'ALESSIO, Sergio, 16026 MONTOGGIO (GE) (IT); REGAZZONI, Stefano, 20146 MILANO (IT)
(74) Representative: Studio Torta S.p.A.

(57) **Abstract**

An assembly for monitoring the emissions of at least one battery (2) is provided with:
• at least one emission collecting head (7), which is provided with an emission inlet (8);
• at least one sensor (10), which is configured to detect the concentration of at least one gas in the collected emissions;
• at least one control device (11), which is configured to process the data acquired by said at least one sensor (10) and to selectively generate signals (S) based on said processing;
• positioning means (13), which are configured to hold the emission inlet (8) at or close to the exhaust (4) of said at least one monitored battery (2).

## Description

### CROSS-REFERENCE TO RELATED APPLICATIONS

This Patent Application claims priority from Italian Patent Application No. 102021000021485 filed on August 6, 2021.

The present invention relates to an assembly and to a method for monitoring the emissions of at least one battery.

In particular, the present invention is particularly usable for monitoring batteries associated with electric motors.

The ever-increasing evolution in the use of electric motors in the automation field, in the industrial tools field and in the automotive field, together with the growing sensibility with regard to the emissions of heat engines has hastened the development and the use of batteries of innovative type.

However, the quickness in the development of batteries does not always coincide with as much quickness in the invention and implementation of systems for mitigating the risks connected to their use and especially to a possible malfunction.

Modern electric batteries with high capacity are generally used by coupling several energy storage cells inside solid protection casings.

During the technical life of a single battery, the pressure and temperature inside the casing are subjected to innumerable variations.

In order to preserve the integrity of the device, a relief valve, normally called "pressure relief" valve, is generally present, preventing critical levels of internal pressure to be reached both in ordinary and extraordinary situations.

Whereas, the heat accumulations at the cells are managed, in the normal operation steps, with dedicated systems (for example pyrolytic graphite sheets) which favour the heat dissipation.

Therefore, during the use, a battery of modern type will be capable of dissipating the heat in excess deriving from the electrochemical transformations which allow the energy storage and, in case of need, of expelling the gaseous component deriving from the ordinary processes.

Therefore, the situation remains under control during the ordinary use.

However, such systems do not ensure the safety of the batteries upon the occurrence of short circuit phenomena inside the single cells.

During the initial steps of such phenomena, in one or more storage cells it is possible to observe a simultaneous heat and pressure increase inside the casing, with the formation of different gases having a non-negligible concentration, deriving both from passages of state and from the degradation of the electrolytes into simpler compounds.

Although the pressure relief valve progressively expels the atmosphere inside the battery, the malfunction often extends to the adjacent cells. This entails a sudden heat and pressure increase up to uncontrollable levels (the so-called thermal runaway phenomenon).

The thermal runaway is normally accompanied by explosions due to the pressure increases not manageable by the systems installed on the casing and by flames fed by the gases produced by the internal reactions.

The explosions of this type, besides causing damage to the battery, can seriously damage the vehicle on which it is installed or the people and the things arranged in the environment surrounding the battery both when the battery is in use on a vehicle or a device, and when it is stored in a dedicated place.

Currently, some solutions of passive type are limited to the use of further safety casings dedicated to containing possible fires or deflagrations.

However, the use of safety casings is an expensive and not very flexible solution because it requires for each battery model to be provided with suitably designed and manufactured casings. Moreover, the casings of this type have important and non-negligible bulks in terms of logistics and storing. Finally, the safety casings cannot be adopted, for example, in batteries already mounted on parked motor vehicles.

Other solutions of active type provide for the use of smoke, heat or in some cases flame detection systems.

However, such methodologies signal the anomaly when the latter has already accomplished its development, even arriving at the thermal runaway phenomenon and unequivocally showing the effects thereof on the outside of its casing. Such situation, also with sudden containment interventions (lowering of the temperature, immersion in water...), in any case puts at risk what is placed very close to the battery. For example, in the places dedicated to the storing of batteries, there is the concrete risk of triggering a non-manageable and seriously risky chain phenomenon.

Finally, some solutions provide for the use of environmental gas detection systems. Such systems are aimed at intercepting the presence of emissions that precede the development of thermal runaway phenomena. However, the systems of this type are usable only in extremely limited volumes, and do not anyway ensure sufficiently prompt reaction times due to the dilution of the gases in the volume in which they are dispersed.

The environmental sensors that intervene at a given threshold will be further influenced by the atmosphere present inside the volume in which the battery is present.

Such limits become even more evident when controlling storage batteries mounted on stationary vehicles. Such systems, in fact, cannot be used in open places, and also in the possible closed rooms it is necessary for the dimensions of the spaces where they are mounted to be very contained and not variable.

In conclusion, modern batteries have risks, whose entity is obviously influenced by their remarkable ability to store energy.

However, the known systems for mitigating said risks with passive and active control are generally not very effective in the view of detecting the triggering and the evolution of a thermal runaway, anyway intervening late.

Therefore, an object of the present invention is to manufacture an assembly for monitoring the emissions of at least one battery which is reliable and usable in all the configurations of use, of storage and of transportation of the batteries.

In accordance with such objectives, the present invention relates to an assembly for monitoring the emissions of at least one battery comprising:
- at least one emission collecting head, which is provided with an emission inlet;
- at least one sensor, which is configured to detect the concentration of at least one gas in the collected emissions;
- at least one control device, which is configured to process the data acquired by said at least one sensor and to selectively generate signals based on said processing;
- positioning means, which are configured to hold the emission inlet at or close to the exhaust of said at least one monitored battery.

In this manner, the active control carried out by the monitoring assembly according to the present invention is independent of the place where the battery is situated. The monitoring assembly, in fact, detects the gas exiting the exhaust and manages to predict the onset of hazardous phenomena, such as the triggering of thermal runaway phenomena. The presence of the positioning means, in fact, ensures that the emission inlet of the emission collecting head is positioned so as to collect the gaseous mixture emitted from the battery before it is dispersed into the environment.

The composition of the gaseous mixture emitted, for example, from the exhaust of the battery, in fact, can be a precursory signal of the passage of the battery from the ordinary operation state to the state of hazardous device for people and things close to it.

The mixture emitted from the exhaust, in fact, is an exhaustive representation of the atmosphere present inside the entire casing in case of anomaly. In the casing, in fact, the volumes available are scarce and are immediately saturated by possible gases deriving from the anomalies of the single cells.

The monitoring assembly according to the present invention is thus capable of promptly predicting the triggering of hazardous and incontrollable situations.

A further object of the present invention is to provide a simple and reliable method for monitoring the emissions of at least one battery.

In accordance with such objects, the present invention relates to a method for monitoring the emissions of at least one battery as claimed in claim 16.

Further characteristics and advantages of the present invention will be clear from the following description of a non-limiting example embodiment thereof, with reference to the figures of the accompanying drawings, wherein:
- Figure 1 is a schematic view, with parts removed for clarity, of the assembly for monitoring the emissions of at least one battery according to the present invention;
- Figure 2 is a schematic perspective view, with parts removed for clarity, of a detail of the monitoring assembly of Figure 1;
- Figure 3 is a schematic perspective view, with parts removed for clarity, of the detail of Figure 2 according to a variation;
- Figure 4 is a schematic view, with parts removed for clarity, of the monitoring assembly according to a first variation of the present invention;
- Figure 5 is a schematic view of the monitoring assembly according to a second variation of the present invention;
- Figure 6 and Figure 7 illustrate details of the variation of the monitoring assembly of Figure 5;
- Figure 8 is a schematic view of the monitoring assembly according to a third variation of the present invention.

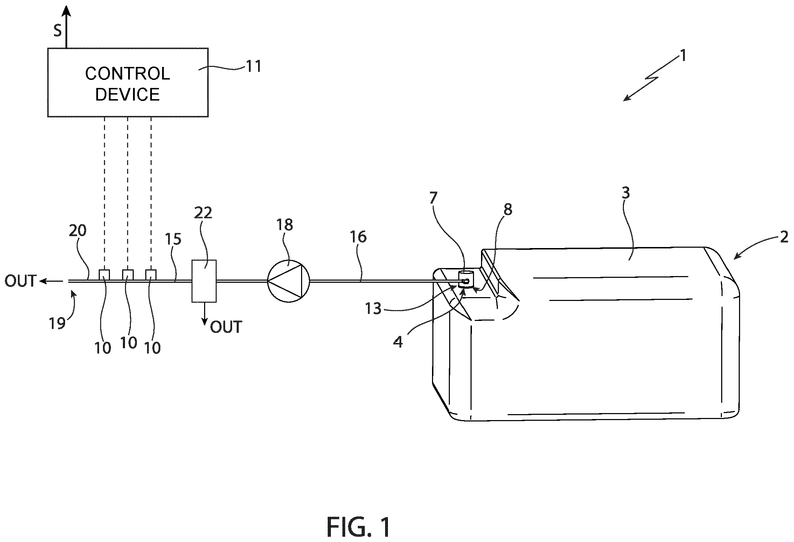

In Figure 1, reference numeral 1 indicates an assembly for monitoring the emissions of a battery 2 according to the present invention.

The battery 2 is provided with an external casing 3, inside which one or more storage cells (not illustrated) are arranged. The external casing 3 is provided with an exhaust 4, configured to discharge the emissions produced by the cells inside the casing 3.

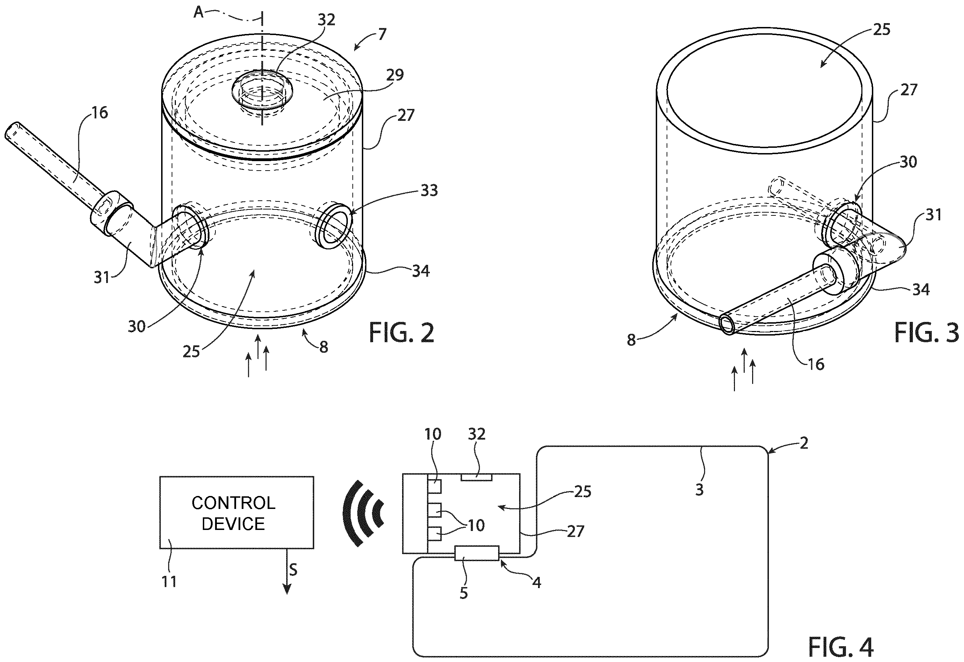

In the non-limiting example described and illustrated herein, the exhaust 4 comprises a pressure relief valve 5 (better visible in Figure 4), which is configured to discharge the emissions accumulated inside the available volume of the casing 3 when said emissions exceed a predetermined pressure level.

The battery 2 illustrated in the accompanying figures is a battery for an electrically powered vehicle, specifically a car. It is understood that the monitoring assembly according to the present invention can also be applied to batteries of different type and different application.

The assembly 1 comprises an emission collecting head 7 which is provided with at least one emission inlet 8, at least one sensor 10 which is configured to detect the presence of at least one gas in the emissions collected by the emission collecting head 7, a control device 11 which is configured to process the data acquired by said at least one sensor 10 and to selectively generate signals S based on said processing, and positioning means 13 which are configured to hold the emission inlet 8 above (i.e. at) or close to the exhaust 4 of the monitored battery 2.

Preferably, the monitoring assembly 1 comprises a plurality of sensors 10, each configured to detect the presence of respective different gases. In particular, the sensors 10 are placed in series so that the emission mixture can be completely analysed.

Each sensor 10 is configured to quantify the concentration of a respective gas that can be representative of the triggering of an anomalous process inside the battery 2.

In the non-limiting example described and illustrated herein, the sensors 10 are three, configured to respectively detect the concentration of carbon monoxide and of carbon dioxide and hydrocarbons. Specifically, the sensors 10 detect a concentration value with respect to the overall emission mixture expressed in Parts per Million or, alternatively, in percentage.

A variation not illustrated provides for the presence of two sensors configured to respectively detect the concentration of carbon monoxide and of carbon dioxide.

A variation not illustrated provides for the presence of only one sensor configured to detect the concentration of carbon monoxide.

A further variation not illustrated provides for the presence of only one sensor configured to detect the concentration of flammable gases. In particular, in this case the sensor will provide a percentage value with respect to the Lower Explosive Limit (LEL) of the mixture in which the flammable gases are present.

A further variation not illustrated provides for the presence of only one sensor configured to detect the concentration of organic compounds with photoionization technology. Such sensor will provide a value expressed in Parts per Billion of the organic compounds present in the emission mixture.

The sensors 10 communicate the detected data to the control device 11. The data can be transmitted with standard communication protocols, via cable or by means of wireless protocols.

The monitoring assembly 1 is further provided with a sampling chamber 15, said at least one sensor 10 facing said sampling chamber 15, a connection pipe 16 which is configured to connect the emission collecting head 7 to the sampling chamber 15, and a pump 18 which is arranged along the connection pipe 16 and is configured to draw the emissions from the emission collecting head 7 and send them to the sampling chamber 15.

The sampling chamber 15 is provided with an exhaust outlet 19, through which the emissions are discharged after the sampling has been carried out by means of the sensors 10.

In the non-limiting example described and illustrated herein, the sampling chamber 15 is defined by a portion of a duct 20.

The pump 18 is preferably activated at regular intervals so that the emissions are fed to the sampling chamber 15 with a given sampling frequency. The activation of the pump 18 is carried out under the control of the control device 11. The sensors 10 are preferably substantially activated with the same frequency, but with a delay such to ensure a correct detection of the data relative to the emissions only when the latter are actually fed to the sampling chamber 15.

A variation not illustrated provides for the pump to be continuously active and for the passage of the emissions in the sampling chamber 15 to be continuous.

Advantageously, the pump 18 ensures that the emissions exiting the exhaust 4 of the battery 2 are suctioned even if the emission collecting head 7 is at a distance from the exhaust 4, as it will be specifically described in the following.

Preferably, the monitoring assembly 1 further comprises a flow rate adjuster 22, which is arranged downstream of the pump 18 and upstream of the sampling chamber 15 so as to adjust the emission flow rate to be fed to the sampling chamber 15 and to allow a correct detection by the sensors 10.

The flow rate adjuster 22 is preferably provided with an exhaust 23 for discharging the emissions when the flow rate of the pump 18 is greater than the flow rate calibrated for the measurement. In this manner, the flow rate in excess is discharged without passing through the sampling chamber 15.

The connection pipe 16 is preferably made of an inert material towards the emissions. In other words, the material of the connection pipe 16 does not react on contact with the emissions.

The pump 18 is preferably a vacuum pump of the membrane type.

With particular reference to Figure 2, the emission collecting head 7 of the non-limiting example described and illustrated herein defines an emission collecting chamber 25.

In order to define the emission collecting chamber 25, the emission collecting head 7 has dimensions that exceed the diameter of the connection pipe 16 to which it is coupled.

The emission collecting head 7 is defined by a hollow body 27 extending along a longitudinal axis A and open at a first end so as to define the emission inlet 8 and closed at a roof 29 at the end opposite the first end along the axis A.

The hollow body 27 is further provided with an outlet 30, preferably made along the lateral wall, so as to put the emission collecting chamber 25 into fluidic communication with the connection pipe 16. Preferably, the outlet 30 is provided with a connector 31 for easing and ensuring a correct and stable connection between the hollow body 27 and the connection pipe 16. The hollow body 27 can have a cylindrical or prismatic, or hemispherical shape.

Preferably, the emission collecting head 7 is provided with a pressure relief valve 32, which is configured to discharge the emissions when the pressure inside the emission collecting chamber 25 exceeds a threshold value. The pressure relief valve 32 is preferably made on the roof 29 of the hollow body 27.

Preferably, the emission collecting head 7 is also provided with a calibrated hole 33 so as to allow the entrance of air and prevent the creation of an excessive depression inside the emission collecting chamber 25.

The calibrated hole 33 is preferably provided with at least one filter (not visible in the accompanying figures). For example, the filter can be an activated carbon filter and/or a molecular filter connected to the sampling head by means of a suitable joint and configured to block the passage of specific gases. Such filter contributes to increasing the reliability of the detection of the monitoring assembly 1. During the use, in fact, the depression created by the pump 18 in the emission collecting chamber 25 can sometimes determine the suction of gaseous components mixed with air through the calibrated hole 33. This can distort the detection for the presence of gases coming from the outside not due to the emissions coming from the exhaust 4.

The emission inlet 8 is surrounded by an edge 34 preferably provided with a gasket (not illustrated in the accompanying examples).

In Figure 3 a variation of the emission collecting head 7 is illustrated, wherein the hollow body 27 is open at both ends. In the non-limiting example described and illustrated herein, the hollow body 27 is substantially a section of a tubular body.

The positioning means 13 are configured to hold the emission inlet 8 in a position such to ensure that the emissions entering through the emission inlet 8 are actually the emissions exiting the exhaust 4 of the monitored battery 2.

Therefore, it is important that the relative position between the emission inlet 8 and the exhaust 4 is held over time ensuring reliability to the measurement carried out by the monitoring assembly 1.

The emission inlet 8 can be positioned above the exhaust 4 or close to the exhaust 4. The suction action given by the presence of the pump 18, in fact, allows arranging the emission inlet 8 also at a given distance from the exhaust 4.

The expression "above the exhaust 4" means that the emission inlet 8 is substantially arranged at the exhaust 4 so as to collect the emissions exiting the exhaust 4 of the battery 2.

In other words, the emission inlet 8 is substantially arranged aligned with the main exiting direction of the gases from the exhaust 4 and at a maximum distance from the exhaust 4 such to allow the collection of at least 80% of the emissions possibly exiting the exhaust 4.

The expression "close to" the exhaust 4 means that the emission inlet 8 can face the exhaust 4 (and is not necessarily aligned with the main exiting direction of the gases from the exhaust 4) at a distance such to allow the collection of at least 80% of the emissions possibly exiting the exhaust 4.

Sometimes, in fact, the exhaust 4 of the battery 2 is not easily accessible, especially when the battery is already mounted, for example, on a motor vehicle.

In the example illustrated in Figure 1, the emission inlet 8 is arranged at the exhaust 4 and, precisely, above the exhaust 4.

The positioning means 13 are thus preferably configured to couple, in a releasable manner, the emission collecting head 7 to the casing 3 of the battery to be monitored or to a structure on the outside of the battery 2, but close to the exhaust 4.

In the example illustrated in Figures 1-4, the positioning means 13 comprise an adhesive material layer, preferably arranged along the edge 34 of the emission inlet 8. Essentially, the edge 34 is glued onto the casing 3 of the battery 2 so that the emission inlet 8 is arranged at the exhaust 4.

A variation not illustrated provides for the adhesive material layer to be applied to a different portion of the emission collecting head 7 so as to couple the emission collecting head 7 to a portion of the casing 3 or to other structures on the outside of the battery 2 and arranged close to the exhaust 4.

A variation not illustrated provides for the positioning means to comprise a mechanical coupling system between the emission collecting head 7 and the exhaust 4 so as to hold the relative positions fixed between the emission collecting head 7 and the exhaust 4. For example, it is possible to use a threaded coupling, a quick coupling of the snap-fit type or a quick hooking to a hooking element, suitably made or already present, on the outer surface of the casing 3.

A further variation provides for the positioning means 13 to comprise magnetic material, applicable to the emission collecting head 7. For example, the edge 34 can be enriched with magnetic material. Such solution is useful when the emission collecting head 7 has to be coupled to elements and structures (for example: battery casing or structures on the outside of the battery close to the battery) made of ferromagnetic material.

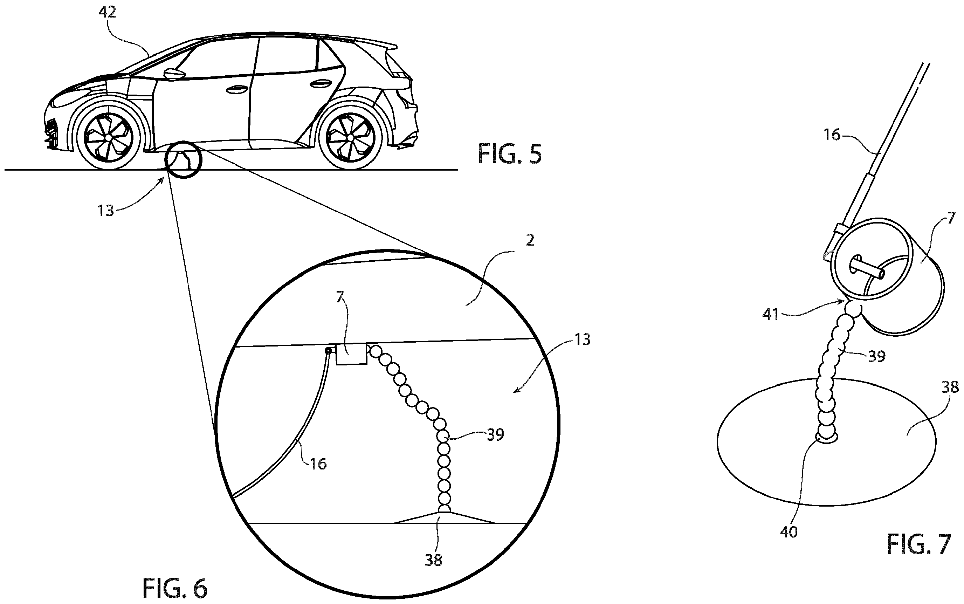

Figures 5-7 illustrate positioning means 13 in accordance with a further variation. In this case, the positioning means comprise a resting base 38 and an adjustable support arm 39, which is provided with a first end 40 connected to the resting base 38 and with a second end 41, opposite the first end 40, coupled to the emission collecting head 7.

The support arm 39 comprises at least one articulated portion for adjusting the position of the emission collecting head 7 so that the emission collecting inlet 8 substantially faces the exhaust 4 at a distance such to allow a suction of at least 80% of the gases possibly exiting the exhaust 4.

A variation not illustrated provides for the support arm 39 to comprise at least one telescopic portion.

In the example of Figures 5-7, the emission collecting head 7 has the structure illustrated in Figure 3.

The solution illustrated in Figures 5-7 can be applied to the monitoring field of the batteries 2 of parked motor vehicles 42 (Figure 5). The positioning means 13, in fact, ease the installation of the emission collecting head 7, exploiting the floor as a rest for the resting base 38.

Figure 4 illustrates a variation of the monitoring assembly according to the present invention wherein the same reference numerals used up to now are kept.

In accordance with such variation, the sensors 10 face the emission collecting chamber 25 and are configured to detect the data and supply them to the control device 11, preferably by means of wireless communication protocols.

In this manner, the structure of the monitoring assembly 1 is reduced to a single body and does not require sampling chambers and suction systems. The overall structure is thus simpler and the installation is eased.

Such solution, however, is particularly suitable to the applications where the monitoring assembly 1 can be arranged at the exhaust 4 or very close to the exhaust 4 so that the emissions exiting the exhaust 4 enter directly in contact with the sensors 10.

The collecting head 7, in this embodiment, will have the structure illustrated in Figure 2 and will preferably be provided with the pressure relief valve 32, so as to prevent the generation in the emission collecting chamber 25 of too high pressures which can also decouple the emission collecting head 7 from the casing 3, and with the calibrated hole 33 so as to prevent depressions inside the emission collecting chamber 25.

Also in this case, it is possible to provide for a variation (not illustrated) wherein the calibrated hole 32 is provided with at least one filter (for example an activated carbon filter and/or a molecular filter configured to block the passage of specific gases).

The control device 11 is configured to process the data coming from the sensors 10 and based on said processing generate one or more signals S.

The signals S can be signals for activating safety devices (for example: fire prevention, flooding, etc.) and/or signals for activating luminous emitters and/or signals for activating acoustic emitters, and/or a signal processable by software, dedicated applications and/or dedicated hardware, for example RFID, GPS and/or independent transmission module connected to the Internet network or in short and/or long range radiofrequency. For example, signals S can be sent to the operating system of the car on which the battery is mounted either by an application in cloud configured to send alerts for example via telephone/sms and/or by means of dedicated supervision apps etc.

In particular, the control device 11 is configured to recognise an alert condition when the sensors 10 detect gas concentrations indicative of the presence of an instability in the battery 2. For example, the indicative conditions can be the exceeding of reference threshold values for periods above a minimum value of the concentrations detected by at least one of the sensors 10.

When the control device 11 recognises an alert condition, it generates the signal S.

Preferably, the control device 11 is also provided with a memory module in which the data acquired, the possible detected alert conditions and the found anomalies are recorded. The control device 11, as already mentioned in part, manages the activation of the pump 18 (if present) and of the sensors 10 based on the established sampling frequency.

For example, in case of activation of the pump 18 at regular intervals (see manufacturing example of Figure 1), the control device 11 is configured to introduce a delay time on the beginning of the detection of the sensor 10 so as to carry out the detection on the emissions just suctioned by the emission collecting head 7 and not on those remained inside the connection pipe 16 at the end of the previous suction cycle.

Therefore, in use, in order to carry out the monitoring of the emissions by means of the monitoring assembly 1 according to the present invention, it will be necessary to position the emission collecting head 7 on the exhaust 4 or close to it depending on the type of positioning means 13 adopted, and to activate the monitoring assembly 1. The control device 11 will thus be capable of autonomously carrying out the entire data acquisition and processing process. The activation of the monitoring assembly 1 entails the activation of the sensors 10 and of the possible pump 18 under the control of the control device 11. In other words, the activation of the monitoring assembly 1 is obtained by means of the activation of at least the control device 11 and the sensors 10.

According to a variation not illustrated, the control device 11 is further capable of monitoring the functioning of the battery correlating the possibly detected gas emissions with the electric behaviour data of the battery collected during the operation of the battery. In this manner, the control device 11 is capable of evaluating the functioning of the battery keeping into consideration the ageing of the battery, the charging and discharging cycles, the duration and suggesting in advance a possible replacement for inefficiency or safety.

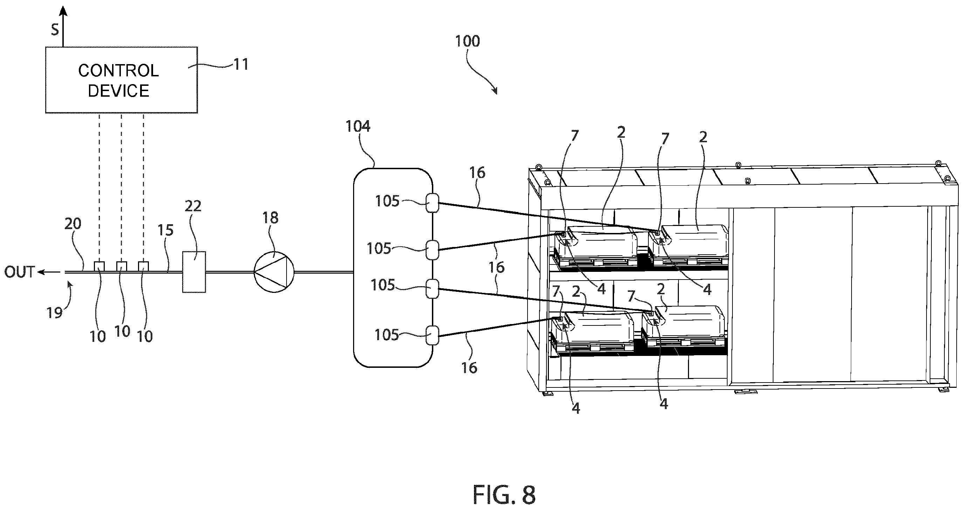

Finally, Figure 8 illustrates a monitoring assembly 100 according to the present invention configured to monitor a plurality of batteries 2. In the non-limiting example described and illustrated herein, the batteries 2 are stored in a dedicated shelf. It is understood that the monitoring assembly 100 can also be used for monitoring batteries mounted on different parked motor vehicles or in other situations in which more than one battery is in stall in a given place.

Here and in the following the same reference numerals adopted in Figures 1-7 will be kept for indicating similar or identical parts.

The monitoring assembly 100 comprises a plurality of emission collecting heads 7 and a plurality of positioning means (not visible) configured to hold the emission inlet 8 of each emission collecting head 7 close to a respective exhaust 4 of the emissions of a respective battery 2 of a plurality of batteries to be monitored.

Also in this case, the expression "close to a respective exhaust 4" means that the emission collecting head 7 is arranged in such a manner that the emission collecting inlet 8 substantially faces the exhaust 4 at a distance such to allow a suction of at least 80% of the gases possibly exiting the exhaust 4.

The monitoring assembly 100 further comprises a plurality of connection pipes 16, an emission manifold 104, a pump 18, a sampling duct 15, a plurality of sensors 10, a control device 11 and a flow rate adjuster 22.

Preferably, the emission manifold 104 is connected to the plurality of connection pipes 16 by means of respective connection valves 105, whose opening is controlled by the control device 11 so that the manifold 104 is supplied by one connection pipe 16 at a time.

Preferably, the valves 105 are solenoid valves directly controlled by the control device 11.

In use, the pump 18 will be continuously active whereas the valves 105, managed by the control device 11, will open cyclically allowing the creation of depression in the different connection pipes 16 and thus in the respective emission collecting heads 7.

Downstream of the pump 18 there will be the same detection structure described in relation to the singlepoint configuration in Figure 1.

The control device 11 acquires the signals coming from the sensors 10 distinguishing them depending on the emission collecting head 7 that carried out the collection. In this configuration, the control device 11 introduces a delay time on the beginning of the analysis so as to carry out the procedures on the emissions just suctioned by the corrected emission collecting head 7 and not on those remained inside the pneumatic circuit at the end of the previous cycle or concerning the sampling of another collecting head 7.

Therefore, in use, in order to carry out the monitoring of the emissions by means of the monitoring assembly 100 according to the present invention it will be necessary to position all the emission collecting heads 7 on the respective exhausts 4 or close to them depending on the type of positioning means adopted, and to activate the monitoring assembly 100. The control device 11 will thus be capable of autonomously carrying out the entire data acquisition and processing process.

Finally, in accordance with the present invention, also a method for calibrating a monitoring device is provided.

The method provides for calibrating said at least one sensor 10 substantially connecting the emission inlet 8 of each emission collecting head 7 to a calibration gas cylinder certified for the zero and span (i.e. known concentration) setting and proceeding with the setting.

Preferably, the step of connecting the emission inlet 8 to the cylinder is carried out by means of a shutter, which is configured to close the emission inlet 8 and is provided with a clutch for the supply duct of the calibration gas coming from the cylinder. In this manner, it is ensured that the sensor 10 carries out detections only on the calibration gas.

The shutter, in fact, simulates the behaviour of the exhaust 4 provided with pressure relief valve 5.

Finally, it is evident that modifications and variations can be made to the assembly and to the method described herein without departing from the scope of the appended claims.

## Claims

1. An assembly for monitoring the emissions of at least one battery (2) comprising:
• at least one emission collecting head (7), which is provided with an emission inlet (8);
• at least one sensor (10), which is configured to detect the concentration of at least one gas in the collected emissions;
• at least one control device (11), which is configured to process the data acquired by said at least one sensor (10) and to selectively generate signals (S) based on said processing;
• positioning means (13), which are configured to hold the emission inlet (8) at or close to the exhaust (4) of said at least one monitored battery (2) .

2. The monitoring assembly according to claim 1, wherein the emission collecting head (7) defines an emission collecting chamber (25).

3. The monitoring assembly according to claim 2, wherein the emission collecting head (7) is provided with a pressure relief valve (32).

4. The monitoring assembly according to claim 2 or 3, wherein said at least one sensor (10) faces the emission collecting chamber (25).

5. The monitoring assembly according to any one of the claims from 1 to 3, comprising:
• a sampling chamber (15), said at least one sensor (10) facing said sampling chamber (15);
• a connection pipe (16), which is configured to connect the emission collecting head (7) to the sampling chamber (15); and
• a pump (18), which is arranged along the connection pipe (16) and is configured to draw the emissions from the emission collecting head (7) and send them to the sampling chamber (15).

6. The monitoring assembly according to claim 5, comprising a flow rate adjuster (22), which is arranged downstream of the pump (18) and upstream of the sampling chamber (15) so as to adjust the emission flow rate to be fed to the sampling chamber (15).

7. The assembly according to claim 5 or 6, wherein the control device (11) is configured to selectively activate the pump (18) with a given sampling frequency.

8. The assembly according to any one of the preceding claims, comprising:
- a plurality of emission collecting heads (7), each provided with a respective emission inlet (8);
- a plurality of positioning means (13), which are configured to hold the emission inlet (8) of each emission collecting head (7) at or close to a respective emission exhaust (4) of a respective battery (2) of a plurality of batteries to be monitored;
- a sampling chamber (15), at least one sampling sensor (10) facing said sampling chamber (15);
- an emission manifold (104);
- a pump (18), which is configured to draw the emissions from the emission manifold (104) and send them to the sampling chamber (15);
- a plurality of connection pipes (16), each configured to connect a respective emission collecting head (7) to the emission manifold (104).

9. The assembly according to claim 8, wherein the emission manifold (104) is connected to said plurality of connection pipes (16) by means of respective connection valves (105), whose opening is controlled by the control device (11) so that the emission manifold (104) is supplied by one connection pipe (16) at a time.

10. The monitoring assembly according to any one of the preceding claims, wherein the positioning means (13) comprise an adhesive material layer applied along at least a portion of the emission collecting head (7).

11. The monitoring assembly according to any one of the claims 1-9, wherein the positioning means (13) comprise a resting base (38) and an adjustable support arm (39), which is provided with a first end (40) connected to the resting base (38) and with a second end (41), opposite the first end (40), coupled to the emission collecting head (7).

12. The monitoring assembly according to any one of the preceding claims, wherein the positioning means (13) are configured to couple, in a releasable manner, the emission collecting head (7) to a casing (3) of the battery (2) to be monitored or to a structure on the outside of the battery (2) close to the exhaust (4) of the battery.

13. The monitoring assembly according to any one of the claims 1-9, wherein the positioning means (13) comprise a magnetic material layer applied to at least a portion of the emission collecting head (7).

14. The monitoring assembly according to any one of the claims from 2 to 13, wherein the emission collecting head (7) is defined by a hollow body (27), which defines the emission collecting chamber (25); the hollow body (27) extending along a longitudinal axis (A) and being open at an end so as to define the emission inlet (8).

15. The monitoring assembly according to claim 14, wherein the hollow body (27) is closed on the side opposite the emission inlet (8) along the axis (A) and is provided with a pressure relief valve (32).

16. A method for monitoring the emissions of at least one battery (2) by means of a monitoring assembly (1; 100) according to any one of the preceding claims, comprising:
• placing said at least one emission collecting head (7) at or close to a respective emission exhaust (4) of said at least one monitored battery (2) ;
• activating the monitoring assembly (1; 100) by means of at least the activation of the control device (11) and of said at least one sensor (10).

17. A calibration method of a monitoring assembly (1; 100) for calibrating the emissions of at least one battery (2) comprising the step of calibrating said at least one sensor (10) by connecting the emission inlet (8) of each emission collecting head (7) to a calibration gas cylinder and proceeding with the setting; wherein the step of connecting the emission inlet (8) to the cylinder is carried out by means of a shutter, which is configured to close the emission inlet (8) and is provided with a clutch for the supply duct of the calibration gas coming from the cylinder.
